Europäisches Patentamt

European Patent Office

Office européen des brevets

(11) Publication number: **0 225 583**
A2

(12) **EUROPEAN PATENT APPLICATION**

(21) Application number: **86116708.8**

(22) Date of filing: **03.12.86**

(51) Int. Cl.⁴: **C 12 P 21/00,** C 07 K 15/00,
C 12 N 5/00, C 12 N 15/00,
C 07 K 3/18, G 01 N 33/577
// (C12P21/00, C12R1:91)

(30) Priority: **03.12.85 JP 272121/85**

(43) Date of publication of application: **16.06.87**
**Bulletin 87/25**

(84) Designated Contracting States: **AT BE CH DE ES FR GB
GR IT LI LU NL SE**

(71) Applicant: **Chugai Seiyaku Kabushiki Kaisha,
5-1, 5-chome, Ukima Kita-ku, Tokyo (JP)**

(72) Inventor: **Nomura, Hitoshi, 3-11-16-218, Igasu,
Suginami-ku Tokyo (JP)**
Inventor: **Imazeki, Ikuo, 1-24-3-401, Magome, Ota-ku
Tokyo (JP)**

(74) Representative: **Vossius & Partner,
Siebertstrasse 4 P.O. Box 86 07 67,
D-8000 München 86 (DE)**

(54) **Monoclonal anti-human granulocyte colony stimulating factor antibody.**

(57) A novel monoclonal anti-human granulocyte sti-
mulating factor (abbreviated as G-CSF) antibody, as well as
a method of purifying human G-CSF by means of using said
antibody and a method of assaying the same with said an-
tibody.
   Said antibody was prepared by the following steps.
   A laboratory animal was immunized eith human G-CSF
derived from an oral cavity cancer serving as an antigen;
   spleen cells were extracted from the animal and fused
with myeloma cells to prepare hybridomas;
   the hybridomas were treated to obtain clones that were
capable of producing a monoclonal anti-human G-CSF antibody in high concentrations; and
   the clones were cultivated to obtain a monoclonal anti-
human G-CSF antibody from the supernatant of the culture.

Our Ref.: W 064 EP
Case: FP(EPC)/C-1-912
Chugai Seiyaku K.K.
Tokyo, JAPAN

VOSSIUS+PARTNER
PATENTANWÄLTE
8000 MÜNCHEN 86
SIEBERTSTRASSE 4 ·
TELEFON 474075

**0 225 583**

2nd December 1986

## MONOCLONAL ANTI-HUMAN GRANULOCYTE COLONY STIMULATING FACTOR ANTIBODY

The present invention relates to a novel monoclonal anti-human granulocyte colony stimulating factor (hereinafter abbreviated as G-CSF) antibody, as well as a method of purifying human G-CSF by means of using said antibody and a method of assaying the same with said antibody.

When bone marrow cells as target cells and kidney cells or fetal cells are cultured by the double-layer soft agar cultivation method, with the bone marrow cells being in the upper layer and the kidney or fetal cells in the lower layer, part of the cells in the upper layer grow and differentiate to form colonies of neutrophilic granulocytes (hereunder simply referred to as granulocytes) or monocytic macrophages. This observation has led to the assumption of the presence in vivo of factors which promote the formation of colonies [Pluznik and Sach, J. Cell. Comp. Physiol., 66, 319 (1965); and Bradley and Metcalf, Aust. J. Exp. Biol. Med. Sci., 44, 287 (1966)].

These factors which are collectively referred to as CSF are known to be produced by cells, such as T-cells, monocytic macrophages, fibroblasts and endothelial cells, which normally are distributed extensively in vivo. Among subclasses of CSF are included: granulocyte-monocytic macrophage CSF (abbreviated as GM-CSF) which act on the stem cells of granulocytes or monocyte macrophages in such a manner that they stimulate the growth of such stem cells and induce their differentiation to form colonies of granulocytes or monocytic macrophages; monocytic macrophage CSF (abbreviated as M-CSF) which is principally capable of forming colonies of monocytic macrophages; multipotent CSF (abbreviated as multi-CSF) which acts on less differentiated multipotent stem cells; and granulocyte CSF (abbreviated as G-CSF) of the type contemplated by the present invention which is principally capable of forming granulocytic colonies. It has recently been held that the stages of differentiation of target cells differ from one subclass of CSF to another [Asano, Taisha - Metabolism and Disease, 22, 249 (1985); and Yunis et al., "Growth and Maturation Factors", edited by Guroff, John Wiley & Sons, NY, vol. 1, 209 (1983)].

Therefore, purifying the individual subclasses of CSF and making closer studies of their chemical and biological properties is very important for the purpose of analyzing the haematopoietic mechanism and pathological aspects of various blood diseases. Two of the biological activities of G-CSF that are attracting the particular attention of researchers are the induction of differentiation of myelocytic leukemia cells and the enhancement of mature granulocytes, and a great hope is seen in the potential clinical utility of human G-CSF in the treatment and prevention of leukemia. Under these circumstances, it is very important to achieve large-scale preparation of human G-CSF in high purity and this has led to an increasing demand for developing an elegant method of isolating and purifying human G-CSF.

The heretofore attempted method of isolating and purifying human G-CSF consists of cultivating human G-CSF producing cells, obtaining the supernatant of the culture, and isolating and purifying the human G-CSF through the steps of fractionation by gel filtration in accordance with the molecular weight and enrichment by ultrafiltration. This method, however, has the problems described below.

As already mentioned, human G-CSF is an endogenous factor that is very interesting not only biologically but also medicinally and it has been strongly desired to develop an efficient isolating and purifying method that is capable of providing human G-CSF of high purity in large volumes.

Conventionally, human G-CSF has been isolated and purified by a method that consists of cultivating human G-CSF producing cells, obtaining the supernatant of the culture, and employing combinations of such steps as the fractionation by gel filtration in accordance with the molecular weight and enrichment by ultrafiltration. However, in order to attain a homogeneous target material, gel filtration by molecular weight must be repeated more than once and the activity of each fraction must be determined for each run. In addition to this complexity of operation, if the target material is an active substance such as CSF, purifying procedures such as gel filtration and ultrafiltration require that there be strict control over the conditions in which they are performed in order to avoid the target material being inactivated during purification.

As a matter of fact, however, human G-CSF is released into the supernatant of the culture in very low concentrations and only traces of human G-CSF can be obtained from a large volume of the culture. Therefore, in order to achieve large-scale preparation of human G-CSF, the scale of the purifying step must be increased appreciably but this is impossible to realize if the purifying step is complicated and requires great care as described above.

It is therefore very important to develop a human G-CSF purifying method that is simple to operate and is suitable for applications where a large volume of the culture is employed. Also important is to develop a method that is capable of simple but precise assaying of human G-CSF.

One object, therefore, of the present invention is to provide a ligand that enables high-purity human G-CSF to be readily obtained in large volumes.

Another object of the present invention is to provide a human G-CSF purifying and assaying method that employs such a ligand.

The present inventors conducted various studies with a view to obtaining high volumes of pure human G-CSF from its solution. As a result, the inventors found that in order to attain the aforementioned objects, it was very effective to use clones that were prepared by the steps of obtaining hybridomas of myeloma cells with the antibody-producing cells from a laboratory animal immunized with human G-CSF, screening and cloning said hybridomas. The present invention has been accomplished on the basis of this finding.

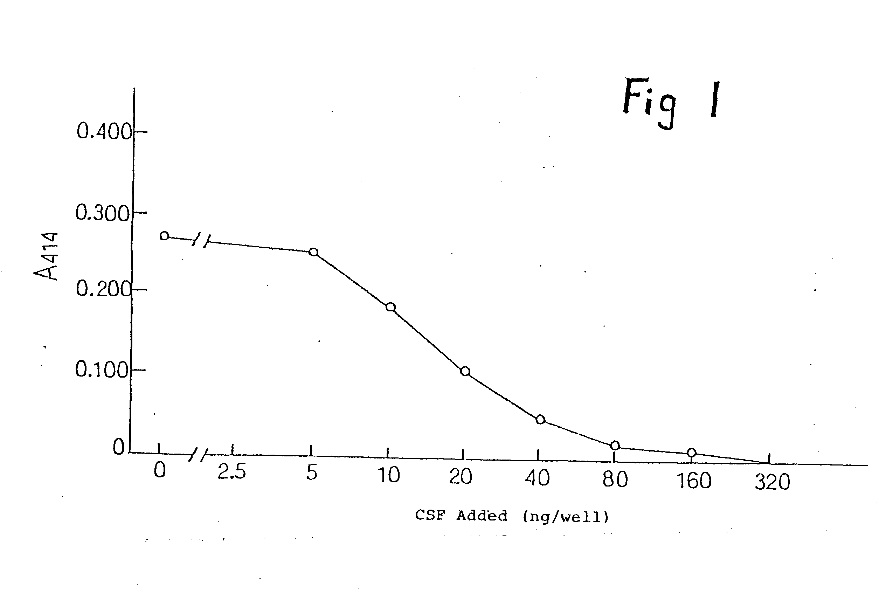

Fig. 1 is a graph showing the profile of absorbance vs the addition of human G-CSF as obtained by drawing a calibration curve for human G-CSF measurement by the antagonistic inhibition method using the monoclonal anti-human G-CSF antibody of the present invention.

The monoclonal anti-human G-CSF antibody of the present invention is novel. A human G-CSF purifying method that relies on affinity chromatography using said monoclonal antibody and an immunological assay of human G-CSF that depends on the use of the same monoclonal antibody are also novel and undocumented in the related literature.

The present inventors immunized a laboratory animal with human G-CSF derived from an oral cavity cancer serving as an antigen; the inventors extracted spleen cells from the animal and fused them with myeloma cells to prepare hybridomas; they then treated the hybridomas to obtain clones that were capable of producing a monoclonal anti-human G-CSF antibody in high concentrations and in a consistent manner [the clones were designated NZ-11 and have been deposited with the Institute for Fermentation, Osaka (IFO) under accession number 50066]. The inventors cultivated these clones and successfully obtained a monoclonal anti-human G-CSF antibody from the supernatant of the culture. This monoclonal anti-human G-CSF antibody reacts immunochemically with human G-CSF derived from the oral cavity cancer; it also reacts immunochemically with the human G-CSF obtained from the cell extract or the supernatant of culture of transformants that were prepared by gene recombinant transformation of E. coli or animal cells with a human G-CSF encoding gene cDNA or chromosomal gene [the two types of gene have been deposited with the Fermentation Research Institute, the Agency of Industrial Science and Technology under accession numbers, FERM BP-954 and FERM BP-955 (cDNA) and FERM BP-956 (chromosomal gene)].

The following steps are essential to the preparation of a monoclonal antibody: (a) preparing a pure biopolymer suitable for use as an antigen; (b) preparing antibody-producing cells by injecting the antigen into a mouse for immunization, taking a blood sample from the animal, and assaying the blood sample in order to determine the proper time for extracting the spleen from the animal; (c) preparing myeloma cells; (d) extracting the spleen and fusing the spleen cells with the myelomas; (e) screening for the hybridomas that produce the desired antibody; (f) dividing the hybridomas into single-cell clones (cloning); (g) optionally cultivating the hybridomas or feeding such hybridoma-transplanted mice in order to achieve large-scale preparation of the monoclonal antibody; and (h) assaying the physiological activity of the so prepared monoclonal antibody or the characteristics of this antibody as a marker reagent.

All of the steps (a) to (h) can be performed in accordance with the conventional method described in "Hybridoma Techniques", Cold Spring Harbor Laboratory, 1980.

In the pages that follow, the method of preparing the monoclonal anti-human G-CSF antibody of the present invention is described in detail according to the individual steps (a) to (h). It should however be noted that antibody-producing cells and myelomas may be obtained from cells other than spleen cells and from mammalian animals other than mouse.

(a)  Obtaining pure antigen:

Human G-CSF is an effective antigen. The present inventors established a cell line, CHU-2, that was capable of specific production of human G-CSF, or a neutrophile colony formation stimulating factor, from tumors in patients with oral cavity cancer who showed a pronounced increase in the number of neutrophiles [this cell line has been deposited with Collection Nationale de Cultures de Micro-organismes (C.N.C.M.) under accession number I-483] and successfully isolated a highly pure product of human G-CSF from the supernatant of the culture of this cell line.

(b)  Preparation of antibody-producing cells:

The supernatant of the culture of CHU-2 capable of specific production of human G-CSF (deposited at C.N.C.M. under accession number I-483) is treated by appropriate methods for separating a pure form of human G-CSF. The human G-CSF is mixed with a Freund's complete or incomplete adjuvant or with another aid such as potassium alum, and the mixture is injected into a laboratory animal as an immunogen.  A BALB/c mouse is preferably used as a laboratory animal for the following reasons:  all of the commonly used mouse myelomas originate from BALB/c mice and their characteristics have been studied fairly well; if both the antibody-producing cells and myelomas originate from a BALB/c mouse, the resulting hybridoma can be grown within the abdominal cavity of the mouse and a monoclonal antibody can be readily obtained from the ascites without requiring any complicated procedures.  It should, however, be remembered that the BALB/c mouse is not the sole laboratory animal that can be used in the present invention.

The immunogen may be administered by any injection method such as subcutaneous injection, intraperitoneal injection, intravenous injection, intradermal injection or intramuscular injection, with subcutaneous or intraperitoneal injection being preferable.

Immunization of the animal may be effected only once or several times at appropriate intervals of, say, one of five weeks.  If desired, the antibody titer of serum from the immunized animal may be measured so as to ensure that the animal has a sufficiently high antibody titer to be suitable for use as a source of the antibody-producing cells, and this is effective in enhancing the efficiency of subsequent steps.  The animal is preferably left alive for 3 - 5 days after final injection so as to obtain antibody-producing cells suitable for use in cell fusion with myeloma cells.

Antibody titer measurement can be achieved by any of the known techniques such as radioimmunoassay (RIA), enzyme-linked immunosorbent assay (ELISA), fluorescent antibody assay and passive hemagglutination assay.  From the viewpoints of sensitivity, rapidity, accuracy and adaptability to automation, RIA and ELISA are preferable.

Antibody titer measurement by ELISA may proceed as follows in the present invention:  the antigen is adsorbed onto a solid phase; any unadsorbed solid surface is covered with a protein such as bovine serum albumin (BSA) that is not related to the antigen; the surface of the solid phase is washed; a first antibody which is made of a serially diluted sample (e.g. mouse serum) is brought into contact with the washed surface so that the antigene is bound with the anti-human G-CSF antibody in the sample; a second antibody which is made of an enzyme-conjugated anti-mouse antibody is added so that it is bound to the mouse antibody; the solid phase is washed again; an appropriate enzyme substrate is added and any resulting change such as color formation that is due to the decomposition of the substrate is analyzed to determine the antibody titer of the sample.

(c)   Preparation of myeloma cells:

Mouse-derived cell lines are preferably used as myeloma cells and they include:  8-azaguanine resistant mouse (BALB/c) myeloma cell line P3-X63 Ag 8-U1 (P3-U1) [Current Topics in Microbiology and Immunology, 81, 1 - 7 (1978)]; P3-NSI/1-Ag 4.1 (NS-1) [European Journal of Immunology, 6, 511 - 519 (1976)]; SP2/O-Ag 14 (SP-2) [Nature, 276, 269 - 270 (1978)]; P3-X63-Ag 8.653 (653) [Journal of Immunology, 123, 1548 - 1550 (1979)]; and P3-X63-Ag 8 (X 63) [Nature, 256, 495 - 497 (1975)].  These cell lines are cultured in appropriate media, such as 8-azaguanine medium [RPMI-1640 medium supplemented with glutamine (1.5 mM), 2-mercaptoethanol ($5 \times 10^{-5}$ M), gentamicin (10 µg/mL), fetal calf serum (FCS, 10%), plus 8-azaguanine], IMDM (Iscove modified Dulbecco's medium) and Dulbecco's MEM.  Three to four days before cell fusion is effected, these cell lines are transferred into normal media so as to ensure at least $2 \times 10^{7}$ cells on the day of cell fusion.

(d)   Cell fusion:

The antibody-producing cells are plasmacytes and lymphocytes which are precursors thereof; these may be obtained from any site of an individual host, typically from the spleen, lymph nodes, peripheral bloods or appropriate combinations thereof; spleen cells are most commonly used.

After final immunization, a site containing antibody-producing cells, say, the spleen, is extracted from mice which have attained the desired antibody titer and spleen cells serving as the antibody-producing cells are prepared. The spleen cells are fused with the myeloma cells obtained in step (c); cell fusion is most commonly performed by means of using a polyethylene glycol (PEG) solution since this produces comparatively low levels of cytotoxicity and achieves the intended fusion in a simple manner. The procedures of cell fusion using a PEG solution are outlined below.

The spleen and myeloma cells are washed thoroughly with an appropriate medium or phosphate buffered saline (PBS), mixed to provide a spleen-to-myeloma ratio of about 5:1 to 10:1, and centrifuged. The supernatant is discarded and the cell-containing precipitate is disintegrated well. To the disintegrated cell precipitate, a mixture of PEG (Mw, 1000 - 4000) and a medium is added under stirring so that $10^3$ spleen cells will exist in 0.1 - 1.0 ml of the solution. Several minutes later, the solution is centrifuged and appropriate amounts of a HBSS solution (Hank's balanced salt solution) and a normal medium containing 20% FCS (e.g. MEM, IMDM or RPMI-1640) are gently added. After disintegrating the cells, the solution is centrifuged and the supernatant is discarded. A HAT medium (a normal medium supplemented with $10^{-6} - 10^{-3}$ M of hypoxanthine, $10^{-8} - 10^{-7}$ M of aminopterin and $10^{-6} - 10^{-4}$ M of thymidine) is added to the cake and the cells are disintegrated again to make a suspension.

(e)   Selection of hybridomas:

The suspension is distributed into wells on an incubation plate and cultivated in a $CO_2$ incubator at 35 - 40°C for 10 - 30 hours. In a subsequent period of 1 - 3 days, half the volume of the supernatant of the culture is discarded every 10 - 30 hours and an equal volume of a fresh HAT medium or any other appropriate medium is added. Thereafter, cultivation is effected in a $CO_2$ incubator at 35 - 40°C for 10 - 14 days.

The myeloma cells used above are 8-azaguanine resistant and hybridomas obtained by fusing two of them with each other are incapable of surviving in a hypoxanthine-aminopterin-thymidine containing medium (HAT medium).

However, hybridomas obtained by fusing the antibody-producing cells with themselves or with myeloma cells are capable of surviving in the HAT medium. In addition, the life of the hybridomas of two antibody-producing cells is limited. Therefore, screening for the desired hybridomas of myeloma and antibody-producing cells can be accomplished by cultivation in a HAT medium.

After identifying the wells containing colonies of the intended hybridomas, half the volume of the supernatant of the culture is discarded and an equal volume of an HT medium (aminopterin-free HAT medium) is added and transfer into an HT medium is repeated every 10 - 30 hours in a subsequent period of 1 - 3 days.

After conducting cultivation in the HT medium for 3 - 4 days, part of the supernatant of the culture is sampled for measuring the anti-human G-CSF antibody titer by an appropriate method such as ELISA.

While the above description assumes the use of an 8-azaguanine resistant cell strain, other cell strains may be employed in accordance with the particular hybridoma to be selected and, in this case, the compositions of the media to be employed should also be varied.

(f)  Cloning:

The hybridomas which have been found to have the ability to specifically produce the intended antibody by titer measurement are transferred onto another plate for achieving their cloning. Cloning can be performed by various methods such as:  limiting dilution wherein dilution is effected in such a manner that one hybridoma is plated in one well; the soft agar method wherein colonies are taken by plating on a soft agar medium; the use of a micromanipulator for delivering and plating one cell at a time; and sorter cloning which employs a cell sorter to separate one cell at a time. Limiting dilution is a simple method and commonly used.

Cloning is repeated two to four times by an appropriate method such as limiting dilution for the wells which have been found to have a predetermined antibody titer and those hybridomas which have been found to have that titer in each run are selected as the anti-human G-CSF monoclonal antibody producing hybridoma cells.

(g)  Preparation of monoclonal antibodies by cultivation of hybridomas:

The cloned hybridomas are cultured on a normal medium which has been changed from the HT medium.  Large-scale cultivation is effected with a whirler or spinner employing a large incubation bottle.  The resulting supernatant is subjected to gel filtration and other appropriate treatments for collecting IgG fractions, which are then purified to obtain anti-human G-CSF monoclonal antibodies.

The hybridomas can also proliferate in the abdominal cavity of a mouse of the same strain as used in the preparation of the antibody-producing cells (e.g. BALB/c mouse) or an Nu/Nu mouse.  For instance, 8 to 10-week old BALB/c female mice are injected intraperitoneally with $2 - 4 \times 10^6$ per animal of the anti-human G-CSF monoclonal antibody producing hybridoma cells; in 10 - 21 days, the hybridomas grow into ascitic cancer; a sample of ascites is taken from each mouse and freed of any solids content by centrifugation; the resulting product can be used as a monoclonal antibody in such applications as the purification and assaying of human G-CSF.

If further purification is necessary, the supernatant obtained by centrifugation may be passed at least once through a column packed with a suitable adsorbent such as DEAE-Sepharose or protein A-Sepharose, followed by collection of IgG fractions.

(h)  Identification of monoclonal antibodies:

The so obtained monoclonal antibodies are classified, or determined for their isotypes and subclasses, by such methods as the Ouchterlony method, ELISA and RIA.  The Ouchterlony method is simple but requires enrichment of the monoclonal antibodies if their concentration is low. In ELISA  or RIA, the supernatant of the culture may be directly reacted with an antigen-adsorbed solid phase and antibodies corresponding to individual IgG subclasses can be used as the secondary antibody.

Protein contents may be determined by the Folin-Lowry method and on the basis of absorbance at 280 nm [1.4 $(OD_{280})$ = 1 μg of immunoglobulin per mL].

As will be described in the Examples that follow, the aforementioned identification and assaying techniques enable one to conclude that the monoclonal antibody produced from the hybridoma having the designation NZ-11 is an isotype of the class IgG and belongs to the subclass $IgG_1$.

The monoclonal antibodies of the present invention have high specificity for human G-CSF and can be prepared in a homogeneous state in high volumes by cultivation of the hybridomas obtained in accordance with the present invention. Therefore, these monoclonal antibodies are useful as ligands in affinity chromatography for the purpose of purifying and recovering human G-CSF.

Affinity chromatography is a very effective technique for the purpose of solely isolating a substance that binds specifically with the support and has the advantage that it requires a very limited number of steps as compared with gel filtration that is applied to a mixture containing said substance. The monoclonal antibodies may be employed as immobilizing media in affinity chromatography in accordance with the methods commonly used in enzyme immobilization, such as one involving the use of a CNBr-activated support. Preferable supports are selected from among the materials commonly employed for this purpose in chromatography and include: cellulose, agarose, cross-linked dextran, polyacrylamide, porous glass, and modifications thereof incorporating a spacer as in Sepharose 4B, Affigel-10 and Biogel.

In the actual operation, human G-CSF can be purified with the immobilizing monoclonal antibody as follows: a solution containing the human G-CSF is passed through a column packed with the monoclonal antibody, whereupon 50 - 70% of the human G-CSF is adsorbed to the column; the column is then eluted with an appropriate solvent such as a glycine-HCl buffer (pH, 2.5), aqueous sodium chloride, propionic acid, dioxane, ethylene glycol, a chaotropic salt, guanidine hydrochloride or urea, whereby human G-CSF is desorbed and obtained as a high-purity product in the effluent.

The monoclonal antibody of the present invention can also be used in assaying human G-CSF by a variety of immunochemical methods. Immunological assays depend on the specificity of an antigen-antibody reaction in which an antibody reacts selectively with the corresponding antigenic substance and they achieve such high levels of detection sensitivity that they are suitable for use in determining the antigenic substance and antibody titer.

As already mentioned, typical methods of immunological assaying include RIA, ELISA, fluorescent antibody assay and passive hemagglutination assay, and ELISA is the most suitable from the viewpoint of various aspects. Human G-CSF may be assayed by ELISA as follows using the anti-human G-CSF monoclonal antibody of the present invention: the specific antibody of the present invention is adsorbed onto a solid phase; that surface of the solid phase to which the antibody has not been adsorbed is covered with a protein that is not related to an antigen of interest; the surface of the solid phase is washed; an enzyme-conjugated antigen and a sample of interest are added to effect antigen-antibody reaction; an enzyme substrate is added and the decrease in absorbance following the addition of the sample is measured to determine the amount of the antigen.

Examples

The following examples are provided for the purpose of further illustrating the present invention but are in no way to be taken as limiting.

In these examples, the CSF activity (hereinafter simply referred to as CSA) was measured by the following methods.

CSA assay

(a) With human bone marrow cells:

Single-layer soft agar cultivation was conducted in accordance with the method of Bradley, T.R. and Metcalf, D.

(Aust. J. Exp. Biol. Med. Sci., 44, 287-300, 1966). More specifically, 0.2 ml of a bovine fetal serum, 0.1 ml of the sample, 0.1 ml of a human bone marrow nonadherent cell suspension ($1 - 2 \times 10^5$ nuclear cells), 0.2 ml of a modified McCoy's 5A culture solution, and 0.4 ml of a modified McCoy's 5A culture solution containing 0.75% of agar were mixed, poured into a plastic dish for tissue culture (35 mm$^{\phi}$), coagulated, and cultured at 37$^{o}$C in 5% $CO_2$/95% air and at 100% humidity. Ten days later, the number of colonies formed was counted (one colony consisting of at least 50 cells) and CSA was determined with one unit being the activity required for forming one colony.

(b)  With mouse bone marrow cells:

A horse serum (0.4 ml), 0.1 ml of the sample, 0.1 ml of a C3H/He (female) mouse bone marrow cell suspension (0.5 $- 1 \times 10^5$ nuclear cells), and 0.4 ml of a modified McCoy's 5A culture solution containing 0.75% of agar were mixed, poured into a plastic dish for tissue culture (35 mm$^{\phi}$), coagulated, and cultured for 5 days at 37$^{o}$C in 5% $CO_2$/95% air and at 100% humidity. The number of colonies formed was counted (one colony consisting of at least 50 cells) and CSA was determined with one unit being the activity for forming one colony.

The modified McCoy's 5A culture solution used in each of the methods (a) and (b) and the human bone marrow nonadherent cell suspension used in (a) were prepared by the following procedures.

(i) <u>Modified McCoy's 5A culture solution (double concentration)</u>

Twelve grams of McCoy's 5A (Gibco), 2.55 g of MEM amino acid-vitamin medium (Nissui Seiyaku Co., Ltd.), 2.18 g of sodium bicarbonate and 50,000 units of potassium penicillin G were dissolved in 500 ml of doubly distilled water and the solution was aseptically filtered through a Millipore filter (0.22 μm).

(ii) <u>Human bone marrow nonadherent cell suspension</u>

A bone marrow fluid obtained from a healthy person by sternal puncture was diluted 5-fold with an RPMI-1640 culture medium, plated over a Ficoll-Paque solution (Pharmacia Fine Chemicals) and centrifuged at 400 x g for 30 minutes at $25^{\circ}C$. The interfacial cell layer (specific gravity <1.077) was recovered. The cells were washed, adjusted to a concentration of 5 x $10^6$ cells/ml with an RPMI-1640 culture medium containing 20% of bovine fetal serum, poured into a 25-$cm^2$ plastic flask for tissue culture, and incubated for 30 minutes in a $CO_2$ incubator. Nonadherent cells were recovered in the supernatant, poured into a plastic flask (25 $cm^2$) and incubated for 2 hours and a half. Nonadherent cells in the supernatant were collected and used in an assay.

Example 1

(a)  Production of an antigen protein (human G-CSF):

The cells established in Example 1 were grown in a completely dense population in two culture flasks (150 $cm^2$). The cells were recovered, suspended in 500 ml of an F-10 culture solution containing 10% of a bovine fetal serum, transferred into a glass roller bottle of 1580 $cm^2$ (Belco), and whirl-cultured at 0.5 rpm. When the cells were found to have grown in a completely dense population on the inner wall of the roller bottle, the culture solution was replaced by a serum-free RPMI-1640. After 4-day culture, the supernatant of the culture was recovered and cultivation was continued with F-10 containing 10% of a bovine fetal serum. After 3-day culture, the culture solution was again replaced by a serum-free RPMI-1640 and the supernatant of the culture was recovered 4 days later. By repeating these procedures, 500 ml of the serum-free supernatant of culture per bottle was obtained each week. In addition, this method enabled the supernatant of culture to be recovered, with the cells maintained over a significantly prolonged period.

A batch consisting of 5,000 ml of the supernatant of the culture obtained was mixed with 0.01% of Tween 20 and concentrated about 1000 times by ultrafiltration with Hollow Fiber DC-4 and Amicon PM-10 (Amicon). The concentrate was purified by the following steps.

(i)    A portion (5 ml) of the concentrated supernatant of culture was subjected to gel filtration on an Ultrogel AcA54 column (4.6 cm$^\emptyset$ x 90 cm$^L$; LKB) at a flow rate of ca. 50 ml/hr with 0.01 M Tris-HCl buffer (pH 7.4) containing 0.15 M NaCl and 0.01% Tween 20 (Nakai Kagaku Co., Ltd.)  The column had been calibrated with bovine serum albumin (Mw; 67,000), ovoalbumin (Mw; 45,000) and cytochrome C (Mw; 12,400). After completion of the gel filtration, 0.1 ml of each of the fractions was diluted 10-fold and screened for the active fractions by the above-described method of CSA assay (b).  The fractions for Ve = 400 - 700 ml were found to exhibit macrophage-dominant CSA while the fractions for Ve = 800 - 1200 ml showed granulocyte-dominant CSA.  Therefore, the latter fractions were collected and concentrated to a volume of ca. 5 ml on an ultrafiltration apparatus with PM-10 (Amicon).

(ii)    To the concentrated fractions was added an aqueous solution of 0.1% trifluoroacetic acid containing 30% of n-propanol (for determination of amino acid sequence; available from Tokyo Kasei K.K.)  After the mixture had been left to stand in ice for about 15 minutes, the precipitate was removed by centrifugation for 10 minutes at 15,000 rpm.  The supernatant was adsorbed on a μ-Bondapak C18 column (8 mm x 30 cm for semipreparatory use; Waters) equilibrated with the aqueous solution containing n-propanol and trifluoroacetic acid; the column was continuously eluted with an aqueous solution of 0.1% trifluoroacetic acid which contained n-propanol having a linear concentration gradient of 30 - 60%. A high performance liquid chromatographic apparatus, Hitachi Model 685-50 (Hitachi, Ltd.), and a detector, Hitachi Model 638-41 (Hitachi, Ltd.) were employed to determine the absorptions at 220 nm and 280 nm simultaneously.  After elution, 10 μl of each of the fractions was diluted 100-fold and screened for the active fractions by the above-described method of CSA assay (b).  The peaks eluted with 40% n-propanol were found to have CSA activity, so they were collected, re-chromatographed under the same conditions, and assayed for CSA by the same method.  Again, CSA activity was observed in the peaks at 40% n-propanol. Therefore, these peaks were collected (4 fractions = 4 ml) and freeze-dried. (iii) The freeze-dried powder was dissolved in 200 µl of an aqueous solution of 0.1% trifluoroacetic acid containing 40% of n-propanol, and the solution was subjected to high performance liquid chromotography on TSK-G 3000SW column (Toyo Soda Manufacturing Co., Ltd.; 7.5 mm x 60 cm). Elution was conducted with the same aqueous solution at a flow rate of 0.4 ml/min and the fractions were taken in 0.4-ml portions with a fraction collector, FRAC-100 (Pharmacia Fine Chemicals). Each of the fractions taken was checked for its CSA by the same method as described above and activity was observed in the fractions for retention times of 37 - 38 minutes (corresponding to MW of ca. $2 \times 10^4$). The active fractions were recovered and purified on an analytical µ-Bondapak C18 column (4.6 mm x 30 cm). The main peaks were recovered and freeze-dried. The sample obtained was assayed by the method of CSA assay (a); it was found to have human G-CSF activity.

The so obtained human G-CSF had a molecular weight of 19,000 ± 1,000 (on SDS-PAGE) and the sequence of the first 21 amino acids from N terminus was as follows:

$H_2N$ - Thr - Pro - Leu - Gly - Pro - Ala - Ser - Ser -
(10)
Leu - Pro - Gln - Ser - Phe - Leu - Leu - Lys - Cys -
(20)
Leu - Glu - Gln - Val -

(b) Preparation of spleen cells from immunized mice:

Three 8-week-old BALB/c female mice were immunized by intraperitoneal injection of 2 mg of an adjuvant (aluminum hydroxide gel) per animal and 20 µg of an antigen [human G-CSF obtained in (a)] per animal.

Subsequent immunization was achieved by administering intraperitoneally 20 µg of human G-CSF per animal at two-week intervals. Five to seven days after each of the third and subsequent immunization, a blood sample was taken from the fundal veins and the titer of the anti-human G-CSF antibody in the serum was measured by ELISA.

<u>ELISA procedures</u>

A specific antigen (a solution of human G-CSF diluted to a concentration of 1 µg/mL with 0.1 M glycine-NaOH buffer having a pH of 9.0) was distributed among 96 holes on an EIA plate (Immunoplate II® of Nunc) so that each well contained 40 µl of the antigen. The plate was left to stand for 2 hours at room temperature so that an antigen coating formed on the bottom of each well. Thereafter, a mixture of 10% FCS/phosphate buffered saline (PBS) was distributed among the wells in amounts of 200 µL/well. The plate was left to stand for 30 minutes at room temperature so that the protein-bindable residue on the bottom of each well was coated with BSA. The plate was washed well with PBS. The first antibody which was a serially diluted sample of the antibody of interest (e.g. mouse serum, the supernatant of the culture of hybridomas, or monoclonal antibody) was distributed among the wells in amounts of 40 µL/well and the plate and left to stand for 2 hours at room temperature. After washing the plate with PBS three times, the second antibody which was a 100-fold dilution of goat anti-mouse IgG bound to peroxidase was distributed among the wells in amounts of 40 µL/well and left to stand for 2 hours at room temperature. After washing the plate with PBS, a solution of substrate for peroxidase [1% $H_2O_2$, 0.1 M acetic acid-0.05 M phosphate buffer, and 2 mM of 2,2'-azino-di-(3-ethylbenzothiazoline sulfate)] was distributed among the wells in amounts of 200 µL/well and left to stand for 10 - 30 minutes at room temperature. Colorimetry was conducted at 414 nm for antibody titer calculation.

After the third and subsequent immunization, all of the three mice were found to have an antibody titer but, in order to ensure efficient production of monoclonal antibodies of the IgG class, further immunization was conducted. In the final step, an additional immunization was conducted by intraperitoneal injection of 20 µg of human-CSF per animal. Three days later, spleen cells were prepared from the immunized mice and used in subsequent cell fusion.

(c)   Preparation of mouse myeloma cells:

P3-U1, or 8-azaguanine resistant mouse myeloma cells, were cultivated in a normal medium (RPMI-1640 supplemented with 1.5 mM of glutamine, $5 \times 10^{-5}$ M of 2-mercaptoethanol, 10 µg/mL of gentamicin, and 0.1 µL/mL of FCS) and, 4 days later, at least $2 \times 10^{7}$ cells were obtained.

(d)   Preparation of hybridomas:

A mixture of $1.2 \times 10^{8}$ spleen cells from the immunized mice and $1.2 \times 10^{7}$ cells of P3-U1 (both types of cells had been washed well with MEM of Nissui Seiyaku Co., Ltd.) was centrifuged at 1,000 rpm for 5 minutes.

The precipitate containing the spleen cells and P3-U1 was disintegrated well and mixed with 2 g of polyethylene glycol 1500 (PEC-1500) and 2 mL of MEM at $37^{\circ}$C with stirring. One minute later, the mixture was centrifuged at 600 rpm for 5 minutes. The cell containing precipitate was disintegrated with 5 ml of HBSS solution and 5 mL of 20% FBS/MEM solution being gently added. One minute later, the precipitate was further centrifuged at 1,000 rpm for 5 minutes and the supernatant was discarded. The cell containing precipitate was mixed with 5 mL of a HAT medium (normal medium supplemented with $10^{-4}$ M of hypoxanthine, $4 \times 10^{-7}$ M of aminopterin and $1.5 \times 10^{-5}$ M of thymidine) so as to form a cell suspension.

(e)   Screening of hybridomas:

The cell suspension was distributed among 24 wells on an incubation plate (product of Flow Laboratory Corporation, U.S.A.) such that each well contained 1 mL of suspension. Thereafter, cultivation was conducted at $37^{\circ}$C for 24 hours in a $CO_2$ incubator (5% $CO_2$ and 95% air). A HAT medium (normal medium supplemented with $10^{-4}$ M of hypoxanthine, $1.5 \times 10^{-5}$ M of thymidine and $4 \times 10^{-7}$ M of aminopterin) was distributed among the wells in amounts of 1 mL/well and cultivation was conducted for an additional 24 hours. After discarding 1 mL of the supernatant of the culture, 1 mL of a fresh HAT medium was added into each well and cultivation was conducted at $37^{\circ}$C for an additional 24 hours. After replacing 1 mL of the supernatant of the culture with an equal amount of a fresh HAT medium, cultivation was conducted at 37°C for 10 - 14 days.

One milliliter of the supernatant of the culture was discarded from those wells which contained fused cells as identified by the growth of colonies. One milliliter of an HT medium (HAT medium without aminopterin) was added into each of these wells and cultivation was conducted at 37°C. For subsequent two days, the cultivation was continued with replacement by an HT medium being effected in a similar manner. Four days later, part of the supernatant of the culture was sampled for determining its anti-human G-CSF antibody titer by ELISA.

(f) Cloning:

Cloning by limiting dilution was conducted twice for the wells which were found to contain a predetermined antibody titer. The clones which were verified to exhibit a predetermined titer in a consistent manner were selected as anti-human G-CSF monoclonal antibody-producing hybridomas.

(g) Partial purification of monoclonal antibodies:

Eight-week old BALB/c female mice were injected intraperitoneally with Pristane (2,6,10,14-tetramethyl pentadecane) in amounts of 0.2 mL per animal and fed for one week. The so treated mice were then injected intraperitoneally with $5 \times 10^6$ hybridoma cells per animal. Ten to twenty-one days later, the hybridomas had grown into ascitic cancer. After an additional 10 - 21 days, ascites (1 - 10 ml) was taken from the cancer-bearing mice and centrifuged to remove any solids content. The supernatant was recovered as a monoclonal antibody.

(h) Assaying the monoclonal antibody:

(i) Antigenic specificity of the monoclonal antibody

Western blotting with a nitrocellulose membrane showed that the monoclonal antibody was specific for human G-CSF irrespective of whether it was the antigen described above or the one produced from transformants with the gene "FERM P-8352", "FERM P-8453" or "FERM P-8454".

(ii)   Classification of the monoclonal antibody

ELISA [see step (b)] showed that the antibody belonged to an $IgG_1$ subclass.

Example 2    Purification of Human G-CSF by Affinity Chromatography

PBS having dissolved therein 1.5 mg of the anti-human G-CSF antibody prepared in Example 1 was reacted with 1 mL of CNBr-activated Sepharose 4B (product of Pharmacia Fine Chemicals) to obtain an immobilizing monoclonal antibody. This antibody was packed in a column through which 20 mL of the supernatant of the culture of CHU-2 cells containing 101 µg of human G-CSF was passed; as a result, 63 µg (ca. 62%) of human G-CSF was adsorbed to the column.

The column was washed with PBS and eluted with a glycine-HCl buffer (pH, 2.5) to obtain 60 µg (95% of the adsorbate) of human G-CSF. This single passage through the column enabled the human G-CSF to be purified by a ratio of ca. 675:1.

Example 3    Enzyme-Linked Immunosorbent Assay

A specific antigen (a solution of human G-CSF diluted to a concentration of 1 µg/mL with 0.1 M glycine/NaOH buffer having a pH of 9.0) was distributed among 96 holes on an EIA plate (immunoplate II® of Nunc) so that each well contained 40 µL of the antigen. The plate was left to stand for 2 hours at room temperature so that an antigen coating would form on the bottom of each well. Thereafter, a solution of 10% FCS/PBS (containing 0.05% Tween and 0.01% Merthiolate) was distributed among the wells in amounts of 200 µL/well. The plate was left to stand for 30 minutes at room temperature so that the protein-bindable residue on the bottom of each well would be coated with serum protein. The plate was washed well with PBS-TM (PBS containing 0.05% Tween and 0.01% Merthiolate). The primary antibody (monoclonal antibody) was distributed among the wells in amounts of 40 µL/well and the plate was left to stand for 2 hours at room temperature. After washing the plate thoroughly with PBS-TM, the secondary antibody which was a 100-fold dilution of peroxidase-conjugated goat anti-mouse IgG (Fc specific) (product of Cappel) was distributed among the wells in amounts of 40 μL/well and left to stand for 2 hours at room temperature.

After thorough washing with PBS-TM, a solution of substrate for peroxidase [1% $H_2O_2$, a 0.1 M acetic acid/0.05 M sodium phosphate buffer, and 2 mM ABTS (2,2'-azino-di-(3-ethylbenzothiazoline sulfate)] was distributed among the wells in amounts of 200 μL/well and left to stand for 10 - 30 minutes at room temperature. Colorimetry was conducted at 414 nm for titer measurement.

The minimum amount of human G-CSF that could be detected by the above-described method was determined by repeating it with the amount of G-CSF coating being varied. The results are shown in accompanying Fig. 1 from which one can see that the detection limit was about 30 ng for $A_{414}$ of down to 0.100. In a modification of this method wherein rabbit anti-mouse IgG and goat anti-rabbit IgG bound to peroxidase were used as secondary and tertiary antibodies, respectively, the detection sensitivity was increased by 10 times and up to about 5 ng of human G-CSF could be detected.

It was possible to perform assaying of human G-CSF in a liquid mixture with contaminants by the following procedures: mixing the primary antibody with the sample so as effect antigen-antibody reaction; washing; adding the secondary antibody and a solution of enzyme substrate; and measuring the decrease in absorbance.

The anti-human G-CSF monoclonal antibody prepared in accordance with the present invention can be used as a ligand in performing affinity chromatography and immunochemical assay of human G-CSF. This enables human G-CSF to be isolated and purified in a simple and precise manner. As a result, the present invention permits the handling of the supernatant of a human G-CSF containing culture in large volumes so as to enable the large-scale preparation of high-purity human G-CSF.

The monoclonal anti-human G-CSF antibody prepared in accordance with the present invention also reacts with the human G-CSF produced from transformants prepared by transforming E. coli or animal cells with a human G-CSF encoding
gene cDNA or chromosomal gene.  Therefore, this antibody is
also useful for the purpose of isolating and purifying the
human G-CSF produced by such transformants.

Claims:

1. A monoclonal anti-human granulocyte colony stimulating factor antibody.

2. A monoclonal antibody according to Claim 1 which belongs to an isotype IgG.

3. A monoclonal antibody according to Claim 2 which belongs to an IgG subclass $IgG_1$.

4. A monoclonal antibody according to any one of the Claims 1 to 3 which is obtained from clones capable of producing said antibody, said clones being prepared by the steps of: forming hybridomas of spleen cells from an immunized mammalian animal with myeloma cells, screening and cloning said hybridomas.

5. A monoclonal antibody according to Claim 4 wherein said mammalian animal is a BALB/c mouse.

6. A method of purifying a human granulocyte colony stimulating factor which comprises the steps of: adsorbing by means of contacting a human granulocyte colony stimulating factor in solution onto an adsorbent which has a monoclonal anti-human granulocyte colony stimulating factor antibody bound to a solid support; eluting the adsorbent; and recovering the human granulocyte colony stimulating factor as the adsorbate.

7. A method according to Claim 6 wherein said solid support is cellulose, agarose, cross-linked dextran, polyacrylamide, porous glass or any one of these supports modified by incorporation of a spacer.

8. A method according to Claim 6 or 7 wherein the adsorbent is eluted with a glycine-HCl buffer solution, a solution of sodium chloride, propionic acid, dioxane, ethylene glycol, a chaotropic salt, guanidine hydrochloride, or urea.

9. A method according to any one of Claims 6 to 8 wherein said monoclonal antibody is obtained from clones capable of producing said antibody, said clones being prepared by the steps of: forming hybridomas of spleen cells from an immunized sensitized mammalian animal with myeloma cells, screening and cloning said hybridomas.

10. A method according to Claim 9 wherein said mammalian animal is a BALB/c mouse.

11. A method of quantitative immunochemical assay of a human granulocyte colony stimulating factor that is achieved by using a monoclonal anti-human granulocyte colony stimulating factor antibody.

12. A method according to Claim 11 which is radioimmunoassay, enzyme-linked immunosorbent assay, fluorescent antibody assay or passive hemagglutination assay.

13. A method according to Claim 11 or 12 wherein said monoclonal anti-human granulocyte colony stimulating factor antibody is obtained from clones capable of producing said antibody, said clones being prepared by the steps of: forming hybridomas of spleen cells from an immunized mammalian animal with myeloma cells, screening and cloning said hybridomas.

14. A method according to any one of the Claims 11 to 13 wherein said mammalian animal is a BALB/c mouse.

Fig 1

CSF Added (ng/well)

0 225 583